(19) Europäisches Patentamt
European Patent Office
Office européen des brevets

(11) Veröffentlichungsnummer: **0 020 789**
**B1**

(12) # EUROPÄISCHE PATENTSCHRIFT

(45) Veröffentlichungstag der Patentschrift: **04.01.84**

(51) Int. Cl.³: **C 07 D 207/267**, C 07 D 211/76, C 07 D 223/10, C 07 B 23/00, C 08 F 26/06

(21) Anmeldenummer: **80900384.1**

(22) Anmeldetag: **01.11.79**

(86) Internationale Anmeldenummer: **PCT/SU 79/00107**

(87) Internationale Veröffentlichungsnummer: **WO 80/00965 (15.05.80** Gazette **80/11)**

(54) **MIT WASSERSTOFF-ISOTOPEN IN ALPHA-POSITION DES LACTAM-RINGS MARKIERTE N-VINYL-LACTAME UND VERFAHREN ZU IHRER HERSTELLUNG.**

(30) Priorität: **04.11.78 SU 2701734**

(43) Veröffentlichungstag der Anmeldung: **07.01.81 Patentblatt 81/1**

(45) Bekanntmachung des Hinweises auf die Patenterteilung: **04.01.84 Patentblatt 84/1**

(84) Benannte Vertragsstaaten: **CH DE FR GB**

(56) Entgegenhaltungen:
**US - A - 3 028 396**

**Houben-Weyl "Methoden der organischen Chemie" Band III, Teil 1 (1955) Seiten 887-8; Wessel "Arzneimittelforschung" 21. Jahrgang, Nr. 10, Seiten 1429-1482**
**New England Journal of Medicine, volume 247, published in 1952 (Boston Massachusetts) Ravin H.A et al. "Polyvinylpyrrolidone as a plasma expander" see pages 921-929**
**Journal of Polymer Science, volume 31, published in 1958 (interscience publishing house New-York) R.S. Gordon "The Preparation of Radioactive Polyvinylpyrrolidone for Medical Use, see pages 191-192**
**Kernenergie, volume 7, published in 1964, (DDR, Berlin) G.T. Uhlenhult et al. "Labelling (Organic compound) with**

(73) Patentinhaber: **VSESOJUZNY NAUCHNO-ISSLEDOVATELSKY INSTITUT TEHNOLOGII KROVEZAMENITELEI I GORMONALNYKH PREPARATOV, Lavrov per. 6, Moscow 109044 (SU)**

(72) Erfinder: **SVERGUN, Vyacheslav Ivanovich, 2-oy Schukinsky Proezd, 2-116, Moscow, 123098 (SU)**
Erfinder: **BAIRAMOV, Jury Juilevich, 4-Y Vyatsky per 20-67, Moscow, 103287 (SU)**
Erfinder: **PANOV, Valery Petrovich, ul. Barklaya, 5/5-30, Moscow, 121096 (SU)**
Erfinder: **KOCHERGIN, Pavel Mikhailovich, Smolensky Bulvar, 2/1-14, Moscow, 119131 (SU)**
Erfinder: **FEDOSEEV, Vladimir Mikhailovich, ul. Veernaya, 3/5-100, Moscow, 119501 (SU)**
Erfinder: **RYAZANTSEV, Georgy Borisovich, Mosc. obl. Naberezhnaya ul. 4-43,,g, Lytkarino, 140061 (SU)**
Erfinder: **KAMAEV, Andrei Vladimirovich, Pervomaiskaya ul. 5/1-30, Moscow, 119211 (SU)**
Erfinder: **EVDAKOV, Vladimir Pavlovich, ul. Marschala Birjuzova, 30-28, Moscow, 123436 (SU)**

(74) Vertreter: **von Füner, Alexander, Dr. et al, Patentanwälte v. Füner, Ebbinghaus, Finck Mariahilfplatz 2 & 3, D-8000 München 90 (DE)**

(56) Entgegenhaltungen: (Fortsetzung)
**3H by using Wilsbach technique", see pages 504-507**
**Collected articles "Reaktsi onnaya sposobnost organicheskikh soedineny", No. 12(1), published in 1975**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 020 789 B1

(56) Entgegenhaltungen: (Fortsetzung)
**(Tartu University publishing house) N.N. Zatsepin et al. "Issledovanie electronnykh vzaimodelstry v pyatichlennykh nasyshchennykh geterotsiklakh metodom osnovnogo deiteroobmena", see pages 193-205**
**I.F. Tupitsvn et al. "IK-spektroskopicheskoe izuchenie elektronnykh vzaimodeistvy v nasyshchennykh geterotsiklakh.", see pages 235-243**

# Mit Wasserstoff-Isotopen in alpha-Position des Lactam-Rings markierte N-vinyl-lactame und Verfahren zu ihrer Herstellung

Die vorliegende Erfindung bezieht sich auf neue chemische Verbindungen - N-Vinyllactame, die mit Wasserstoffisotopen in der $\alpha$-Stellung in bezug auf die Carbonyl-Gruppe des Ringes markiert sind, sowie auf ein Verfahren zu deren Herstellung. Die obengenannten Verbindungen stellen Ausgangsprodukte zur Synthese der Homo- oder Copolymere dar, welche in der Medizin als diagnostische Mittel sowie für wissenschaftliche Foschungszwecke verwendet werden können.

*Vorhergehender Stand der Technik*

Es ist ein Verfahren zur Herstellung von mit Wasserstoffisotopen markierten gesättigten heterocyclischen Verbindungen durch Austauschreaktion im wasserfreien Medium in Anwesenheit eines alkalischen Katalysators bekannt. So geht im $D_4$-Methanol- und $D_3$-Natrium-methylat-Medium ein Austausch der beweglichen Wasserstoffatome in der $\alpha$-Stellung des Lactamringes von 2-Pyrrolidon gegen Deuteriumatomen vor sich [N.N. Sazepina, I.F. Tupizyn u.a, «Untersuchung der elektronischen Wechselwirkungen in fünfgliedrigen gesättigten Heterozyklen mittels einer basischen Deuteroaustauschmethodik», in «Reaktionsfähigkeit organischer Verbindungen», Nr. 12 (1), Sammlung herausgegeben von der Universität Tartu, 1975, S. 193 bis 205; I.F. Tupisyn, N.N. Sazepina, N.S. Kolodina, A.I. Beljaschova, «IR-spektrometrische Untersuchung der elektronischen Wechselwirkungen in gesättigten Heterozyklen», ebenda, S. 235-243].

Die Herstellung der Endprodukte nach dem bekannten Verfahren ist jedoch unmöglich, da N-Vinyllactame in der Austauschreaktion in einem wasserfreien Medium nicht teilnehmen.

Die in der $\alpha$-Stellung mit Deuterium bzw. Tritium markierten N-Vinyllactame und Verfahren zu ihrer Herstellung sind in der Literatur nicht beschrieben.

*Offenbarung der Erfindung*

Der Erfindung wurde die Aufgabe zugrundegelegt solche neue Substanzen — in der $\alpha$-Stellung des Lactamringes mit Wasserstoffisotopen markierter N-Vinyllactame — herzustellen, welche als Ausgangsstoffe zur Synthese der in der $\alpha$-Stellung des Lactamringes markierten hochmolekularen Verbindungen dienen, die zur Verwendung in der Medizin sowie für wissenschaftliche Forschungszwecke geeignet sind.

Diese Aufgabe wird dadurch gelöst, dass erfindungsgemäss neue Verbindungen vorgeschlagen werden, nämlich-N-Vinyllactame, die mit Wasserstoffisotopen in der $\alpha$-Stellung des Lactamringes markiert sind und die folgende Formel aufweisen:

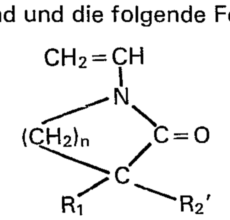

worin n = 2 bis 4, $R_1$ für H, D, T; $R_2$ für D, T steht.

Es wird ausserdem erfindungsgemäss ein Verfahren zur Herstellung der obengenannten Substanzen vorgeschlagen, das darin besteht, dass man das Ausgangs-N-Vinyllactam mit Deuteriumoxid oder mit tritiumenthaltendem Wasser bei einem pH-Wert von 10,5 bis 14 unter Verwendung als Katalysator eines Alkalimetallhydroxides oder einer quaternären Base $NR_4OH$, worin R für $CH_3$ oder $C_2H_5$ steht, in Anwesenheit eines organischen Lösungsmittels oder ohne dieses bearbeitet und ausschliessend das Endprodukt isoliert.

Die vorliegende Erfindung ermöglicht die Herstellung der in der $\alpha$-Stellung des Lactamringes mit den Wasserstoffisotopen markierten N-Vinyllactame mit einem beliebigen Substitutionsgrad von Wasserstoff durch Deuterium oder mit einer beliebigen erforderlichen Radioaktivität bei der Substitution von Wasserstoff durch Tritium. Das Verfahren zur Herstellung von markierten N-Vinyllactamen ist technologisch einfach und wird mit der Bildung von Nebenprodukten nicht kompliziert, da beispielsweise das N-Vinylpyrrolidon in Anwesenheit von Alkalimetallhydroxidlösungen stabilisiert wird.

Die gewonnenen Substanzen haben solche physikalisch-chemische Konstanten, die sich von den Konstanten derselben mit Wasserstoffisotopen nicht markierten Verbindungen praktisch nicht unterscheiden.

Die Strukturidentität der N-Vinyllactame in den H-, D- und T-Formen wird durch Vergleich der Spektra der magnetischen Kernresonanz NMR-$^1H$ und NMR-$^{13}C$ bestätigt.

Die Stellung der Markierung im Falle der Substitution von Wasserstoff durch Deuterium beispielsweise für N-Vinylpirrolidon wird nach dem NMR-$^{13}C$-Spektrum dieser Verbindung mit breitbandiger Protonenentkopplung festgestellt. Bei der H-Form werden die Resonanzen der Kohlenstoff-13-Atom-kerne der Gruppe -$^{13}C$=O bei $\delta$ = 176,9 ppm, der Gruppe -N-$^{13}CH$= bei $\delta$ = 129,2 ppm, der Gruppe =$^{13}CH_2$ bei $\delta$ = 97,8 ppm, der Gruppe -$^{13}CH_2$-C=O des Ringes bei $\delta$ = 32,1 ppm, der Gruppe -C-$^{13}CH_2$-C- des Ringes bei $\delta$ = 17,6 ppm, und der Gruppe -N-$^{13}CH_2$- des Ringes bei $\delta$ = 46,2 ppm beobachtet. Substitution von H durch D in der Methylengruppe des Ringes -$^{13}CH_2$-C=O führt zu einer Änderung der chemischen Verschiebungen der Signale entsprechender Kohlenstoffatomkerne in der Richtung des stärkeren Feldes infolge des Isotopeneffektes für -$^{13}CHF$-C=O um 0,3 ppm, für -$^{13}CH_2$-C=O um 0,6 ppm, für -$^{13}CH_2$-CHD-C=O um 0,1 ppm, für -$^{13}CH_2$-$CD_2$-C=O um 0,2 ppm und zur Multiplettaufspaltung im NMR-$^{13}C$-Spektrum mit breitbandiger Protonenentkopplung, wodurch wegen des Vorhandenseins einer Spin-Spin-Wechselwirkungskonstante $^1J_{CD}$ für $^{13}CHD$-Gruppe ein 1:1:1-Triplett mit $^1J_{CD}$ = 20,4 Hz und für $^{13}CD_2$-Gruppe ein 1:2:3:2:1-Quintett mit $^1J_{CD}$ = 20,4 Hz entsteht. Die Intensität der Signale der $^{13}CD_2$-Gruppe im Spektrum ist infolge des Fehlens des Overhausereffekts

im Falle der Resonanz der Atomkerne der genannten Gruppe vermindert.

In einem NMR-$^{13}$-C-Spektrum von N-Vinylcaprolactam (Resonanzen der Kohlenstoff-13-Atomkerne: für $-^{13}C{=}O$ bei $\delta = 177{,}1$ ppm, für $={}^{13}CH-$ bei $\delta = 132{,}2$ ppm, für $={}^{13}CH_2$ bei $\delta = 95{,}3$ ppm, für $-^{13}CH_2-N-$ bei $\delta = 44{,}9$ ppm, für $-^{13}CH_2-C{=}O$ bei $\delta = 37{,}0$ ppm, für $-^{13}CH_2-CH_2-CH_2-C{=}O$ bei $\delta = 23{,}5$ ppm) werden ähnliche Veränderungen bei der Substitution von H in der Methylengruppe des Ringes $-^{13}CH_2-C{=}O$ durch D beobachtet (Signal der Gruppe $-^{13}CHD-C{=}O$: ein 1:1:1-Triplett, $^1J_{CD} = 20{,}4$ Hz, Isotopenverschiebung $\Delta = 0{,}3$ ppm; Signal der Gruppe $-^{13}CD_2-C{=}O$: ein 1:2:3:2:1-Quintett, $^1J_{CD} = 20{,}4$ Hz; für $-^{13}CH_2-CHD-C{=}O$ $\Delta = 0{,}1$ ppm; für $-^{13}CH_2-CD_2-C{=}O$ $\Delta = 0{,}2$ ppm). Die chemischen Verschiebungen in den NMR-$^{13}$-C-Spektra sind in bezug auf Tetramethylsilan (TMS) gemessen.

Der Isotopenaustauschgrad beispielsweise im N-Vinylpyrrolidon wird im Falle der Substitution von Wasserstoff durch Deuterium mittels Anwendung der NMR-$^1$H-Methodik nach dem Verhältnis der integralen Intensitäten der Signale der Methylenprotonen der Gruppe $-CH_2-C{=}O$ des Ringes ($\delta = 2{,}4$ ppm) zur Intensität der Signale der Methylenprotonen der Gruppe $-N-CH_2-$ des Ringes ($\delta = 3{,}6$ ppm) bzw. der Protonen der Vinylgruppe $-CH{=}CH_2$ ($\delta = 6{,}9$ und $4{,}7$ ppm) eingeschätzt. Eine ähnliche Bewertung für N-Vinylcaprolactam wird nach den Signalen der Methylenprotonen der Gruppen des Ringes $-CH_2-C{=}O$ ($\delta = 2{,}5$ ppm), $-N-CH_2-$ ($\delta = 3{,}5$ ppm), $-C-CH_2-C-$ ($\delta$ 1,5 ppm) sowie der Protonen der Vinylgruppe $-CH{=}CH_2$ ($\delta = 6{,}9$ und $4{,}5$ ppm) erfüllt. Chemische Verschiebungen im NMR-$^1$H-Spektrum sind in Beziehung auf den inneren Standard (4,4-Dimethyl-4-silapentannatriumsulfonat) gegeben.

Der Isotopenaustauschgrad von Wasserstoff durch Tritium wird durch Messung der spezifischen Radioaktivität der Substanz mittels eines Szintillationszählers festgestellt.

Das Verfahren zur Herstellung der Verbindungen der obenangeführten allgemeinen Formel besteht im folgenden. Man bringt in einen Reaktor N-Vinyllactam, Deuterium- bzw. Tritiumoxid und als Katalysator Alkalimetallhydroxid oder quaternäre Base $NR_4OH$, worin R für $CH_3$ oder $C_2H_5$ steht, ein. Der Prozess wird bei einem pH-Wert von 10,5 bis 14 durchgeführt. Als Alkalimetallhydroxide kann man beispielsweise KOH, NaOH, LiOH, CsOH verwenden.

Im Falle der Verwendung von N-Vinylcaprolactam wird der Prozess mit Zusatz von einem mit Wasser mischbaren Lösungsmittel durchgeführt, um das Auftreten einer Phasengrenzfläche zu vermeiden. Als organisches Lösungsmittel verwendet man beispielsweise Methanol, Äthanol, Dimethylformamid, Dioxan oder Aceton.

Die Umsetzung der obenangeführten Reaktionsstoffe kann man bei einer Temperatur in einem Bereich vom Gefrierpunkt bis zum Siedepunkt des Reaktionsgemisches verwirklichen.

Die N-Vinyllactame kann man aus dem Reaktionsgemisch auf eine beliebige bekannte Weise ausscheiden, beispielsweise durch Extraktion mit einem organischen Lösungsmittel, das mit Wasser unmischbar ist. Nach dem Isolieren des Endproduktes führt man eine Analyse dieses Produktes durch. Der Substitutionsgrad von Wasserstoff durch Deuterium wird mittels der NMR-Methodik bestimmt. Der Isotopenaustausch von Wasserstoff durch Tritium wird radiometrisch festgestellt.

*Die beste Ausführungsvariante der Erfindung*

Das bevorzugte Verfahren zur Herstellung der mit Wasserstoffisotopen in der $\alpha$-Stellung des Lactamringes markierten N-Vinyllactame besteht im folgenden.

Man bringt in den Reaktor das Ausgangs-N-Vinyllactam mit Deuteriumoxid beziehungsweise tritiumenthaltendem Wasser ein. Es ist ein Komponentenverhältnis von 1:3 bis 1:20 bevorzugt. Danach bringt man eine gewisse Menge des Katalysators ein, so dass der pH-Wert des Mediums zwischen den Grenzwerten von pH = 13 bis pH = 14 liegt. Als Katalysator verwendet man Natrium- oder Kaliumhydroxid. Nach den obengenannten Operationen wird das Reaktionsgemisch erwärmt. Es ist bevorzugt in einem Temperaturenbereich von 80 bis 100°C zu arbeiten, weil die Reaktionsdauer durch Steigerung der Reaktionsgeschwindigkeit des Austausches vermindert wird und von 15 bis 40 min beträgt.

Im Falle, wo ein höherer Substitutionsgrad von Wasserstoff durch Deuterium notwendig ist, wird das nach der Extraktion des markierten N-Vinyllactames und dem Abdestillieren des Lösungsmittel erhaltene markierte N-Vinyllactam zum zweitenmal dem obenbeschrieben Prozess bei denselben Bedingungen unterworfen.

Zu einem besseren Verständnis der vorliegenden Erfindung führen wir folgende Beispiele ihrer Verwirklichung an.

*Beispiel 1*

Man fügt 0,111 g N-Vinylpyrrolidon zu einer Lösung von 0,5 g Natriumhydroxid in 2 ml $D_2O$ hinzu und hält das Gemisch bei einer Temperatur von 4°C im Verlaufe von 48 Stunden. Um dann N-Vinylpyrrolidon herauszuziehen, bearbeitet man mehrfach das Reaktionsgemisch mit Diäthyläther (fünfmal je 10 ml). Dann vereinigt man die Ätherauszüge, trocknet sie über $MgSO_4$ und entzieht den Äther in einem Rotorverdampfungsapparat. Die Ausbeute an deuteriertem N-Vinylpyrrolidon beträgt 0,1 g (90%). Der Substitutionsgrad von Wasserstoff in der $\alpha$-Stellung des Ringes durch Deuterium, bewertet nach dem NMR-$^1$H-Spektrum, beträgt 50%.

*Beispiel 2*

Man fügt 0,111 g N-Vinylpyrrolidon zu einer Lösung von 0,5 g Natriumhydroxid in 2 ml $D_2O$ hinzu. Man hält das Gemisch im Verlaufe von 2 Stunden bei einer Temperatur 50°C und dann scheidet das Produkt analog dem Beispiel 1 aus. Die Ausbeute an deuteriertem N-Vinylpyrrolidon beträgt 0,1 g (90%), der Substitutionsgrad von Wasserstoff in der $\alpha$-Stellung des Pyrrolidonringes durch Deuterium - 90%.

*Beispiel 3*

Man führt das Einbringen der Reaktionsstoffe und den Prozess analog dem Beispiel 1 durch mit der Ausnahme, dass die Reaktionstemperatur 95°C und die Reaktionsdauer 20 min beträgt. Die Ausbeute an deuteriertem N-Vinylpyrrolidon beträgt 0,1 g (90%), der Substitutionsgrad von Wasserstoff in der $\alpha$-Stellung durch Deuterium - 90%.

*Beispiel 4*

Man fügt 0,222 g N-Vinylpyrrolidon zu 1 ml einer Lösung von $N(C_2H_5)_4OD$ in $D_2O$ (pH = 13,4) hinzu, erwärmt das Reaktionsgemisch im Verlaufe von 40 Minuten bei einer Temperatur von 60°C. Das Produkt scheidet man aus, wie im Beispiel 1 beschrieben ist. Die Ausbeute an deuteriertem N-Vinylpyrrolidon beträgt 0,205 g (92,7%), der Substitutionsgrad von Wasserstoff in der $\alpha$-Stellung durch Deuterium - 90%.

Die erhaltene Verbindung kann man bei der Synthese des in der $\alpha$-Stellung deuterierten Poly-N-vinylpyrrolidons verwenden.

Zu einer Lösung, die 1,4 g in der $\alpha$-Stellung deuteriertes N-Vinylpyrrolidon, welches wie oben angegeben gewonnen wurde, 3,5 g distillertes Wasser enthält, fügt man 0,08 g 30%iges Wasserstoffperoxid und 0,03 g 25%ige wässerige Ammoniaklösung hinzu. Man rührt das Gemisch um und bringt es in eine Glasampulle hinein. Man verlötet danach die Ampulle und polymerisiert das Gemisch im Verlaufe von 3 Stunden bei einer Temperatur von 70°C. Dann öffnet man die Ampulle, verdampft das Wasser, löst die Polymerisationsmasse in Äthanol und fällt sie mit Diäthyläther aus. Der weisse Niederschlag wird an einem Filter abgepresst und im Vakuum bei einer Temperatur von 50°C getrocknet. Man erhält 1,2 g deuteriertes Poly-N-vinylpyrrolidon (86%) bei einem Substitutionsgrad von Wasserstoff in der $\alpha$-Stellung durch Deuterium 90%. Die Grundviskosität beträgt 0,095 dl/g ($M_w$ = 14000).

Der Substitutionsgrad von Wasserstoff durch Deuterium wird nach dem Verhältnis der Integralintensitäten der Signale der NMR$^1$H-Spektra in den Bereichen von $\delta$ 2 ppm und 4 ppm bestimmt.

Die Strukturidentität der Poly-N-vinylpyrrolidone in den H- und D-Formen wird durch Vergleich ihrer NMR-$^1$H- und NMR-$^{13}$C-Spektra bestätigt.

In den NMR-$^{13}$C-Spektra der Lösungen von Poly-N-vinylpyrrolidon in H- und D-Formen werden die Resonanzsignale der Kohlenstoffatomkerne aus der Hauptkette der Polymere unabhängig vom Isotopensubstitutionsgrad im Bereich der $\delta$-Werte 46,5 - 45,1 ppm für die Gruppe $-N-^{13}CH=$ und der $\delta$-Werte 36,1 - 33,0 ppm für die Gruppe $-^{13}CH_2-CH-$ beobachtet. Dieselben Spektra bestätigen die Isotopensubstitution in der $\alpha$-Stellung in bezug auf die Carbonylgruppe des Ringes aufgrund des Vorhandenseins einer vom Isotopeneffekt verursachten Änderung $\Delta$ = 0,6 ppm der chemischen Verschiebung des Signals $\delta$ = 31,5 ppm der Gruppe $-^{13}CD_2-C=O$ im NMR-$^{13}$C-Spektrum mit breitbandiger Protonenkopplung sowie aufgrund der verminderten Intensität dieses Signals infolge des Fehlens des Overhausereffektes für die Resonanz der Kohlenstoffatomkerne der Gruppe $^{13}CD_2$.

*Beispiel 5*

Man fügt 0,111 g N-Vinylpyrrolidon und 0,3 ml $d_6$-Aceton zu einer Lösung von 0,15 g Cäsiumdeuteroxid in 1 ml $D_2O$ hinzu. Das Gemisch erwärmt man in einer verlöteten Ampulle im Verlaufe von 15 Minuten bei einer Temperatur von 100°C. Man scheidet das Produkt analog dem Beispiel 1 aus. Die Ausbeute an deuteriertem N-Vinylpyrrolidon beträgt 0,1 g (90%), der Substitutionsgrad von Wasserstoff in der $\alpha$-Stellung durch Deuterium - 95%.

*Beispiel 6*

Man fügt 2,5 g N-Vinylpyrrolidon zu einer Lösung von 0,15 g Kaliumhydroxid in 7,5 ml tritiertem Wasser mit einer spezifischen Radioaktivität 0,77 Ci/ml hinzu, hält das Gemisch im Verlaufe von 2 Stunden bei einer Temperatur von 70°C; dann wird das Produkt analog dem Beispiel 1 ausgeschieden. Die Ausbeute an tritiertem N-Vinylpyrrolidon beträgt 2,4 g (96%), die spezifische Radioaktivität - 28,2 mCi/g.

Das erhaltene in der $\alpha$-Stellung tritierte N-Vinylpyrrolidon wird bei der Synthese des in der $\alpha$-Stellung tritierten Poly-N-vinylpyrrolidons Anwendung finden.

Man bringt in eine Ampulle nacheinander 1,5 g in der $\alpha$-Stellung tritiertes N-Vinylpyrrolidon, das wie oben angegeben erhalten ist und eine spezifische Radioaktivität von 28,2 mCi/g aufweist, 3,5 g Isopropylalkohol und 0,02 g Azo-bis-isobutyronitril ein. Dann verlötet man die Ampulle und polymerisiert das Gemisch im Verlaufe von 4 Stunden bei einer Temperatur von 70°C. Danach öffnet man die Ampulle, fügt 5 g Isopropylalkohol hinzu und fällt die Polymerisationsmasse mit Diäthyläther aus. Den Niederschlag presst man an einem Filter ab, trocknet im Vakuum bei einer Temperatur von 50°C aus und man erhält das tritierte Poly-N-vinylpyrrolidon mit einer spezifischen Radioaktivität 28,2 mCi/g als weisses Pulver. Die Ausbeute an dem Polymer beträgt 1,4 g (94%). Die Grundviskosität der Probe beträgt 0,19 dl/g ($M_w$ = 30000).

Die Strukturidentität des Poly-N-vinylpyrrolidons in den H- und T-Formen wird durch Vergleich ihrer NMR-$^{13}$C-Spektra bestätigt. Der Isotopensubstitutionsgrad wird radiometrisch festgestellt.

Es ist möglich, das in der $\alpha$-Stellung tritierte Poly-N-vinylpyrrolidon mit einer höheren molekularen Masse zu erhalten.

Dazu bringt man in eine Ampulle 1,5 g in der $\alpha$-Stellung tritiertes n-Vinylpyrrolidon, 3,6 g Äthylalkohol und 0,005 g Azo-bis-isobutyronitril ein. Das Gemisch wird in einer verlöteten Ampulle im Verlaufe von 6 Stunden bei einer Temperatur von 70°C polymerisiert. Danach öffnet man die Ampulle, fügt 5 g Äthylalkohol hinzu und fällt die Polymerisationsmasse mit Diäthyläther aus. Das Polymer wird an einem Filter abgepresst und getrocknet. Man erhält 1,35 g (90%) tritiertes Poly-N-Vinylpyrrolidon mit einer spezifischen Radioaktivität 28,2 mCi/g. Die Grundviskosität der Probe beträgt 0,26 dl/g ($M_w$ = 50000).

*Beispiel 7*

Man fügt 0,139 g N-Vinylcaprolactam in 0,8 ml $CD_3OD$ zu 1 ml einer Lösung von $N(C_2H_5)_4OD$ in $D_2O$, (pH = 13,4) hinzu. Man erwärmt das Gemisch in Verlaufe von 2 Stunden in einer verlöteten Ampulle

bei einer Temperatur von 80°C. Das Produkt scheidet man aus, wie im Beispiel 1 beschrieben ist. Die Ausbeute an deuteriertem N-Vinylcaprolactam beträgt 0,12 g (87%), der Substitutionsgrad von Wasserstoff in der α-Stellung des Ringes durch Deuterium - 50%.

*Beispiel 8*

Man fügt 0,139 g N-Vinylcaprolactam in 0,8 ml $CD_3OD$ zu einer Lösung von 0,4 g Kaliumhydroxyd in 1 ml $D_2O$ hinzu. Man erwärmt das Gemisch im Verlaufe von 1,5 Stunden bei einer Temperatur von 100°C. Das Produkt scheidet man analog dem Beispiel 1 aus. Die Ausbeute an deuteriertem N-Vinylcaprolactam beträgt 0,12 g (87%), der Substitutionsgrad von Wasserstoff in der α-Stellung durch Deuterium ist gleich 90%.

*Beispiel 9*

Man fügt 1,39 g N-Vinylcaprolactam in 1 ml Methanol zu einer Lösung von 0,15 g Kaliumhydroxid in 7,5 ml tritiertem Wasser mit einer spezifischen Radioaktivität 0,77 Ci/ml hinzu. Man erwärmt das Reaktionsgemisch im Verlaufe von 4 Stunden bei einer Temperatur von 100°C. Das Produkt scheidet man analog dem Beispiel 1 aus. Die Ausbeute an tritiertem N-Vinylcaprolactam, das eine spezifische Radioaktivität 25,1 mCi/g hat, beträgt 1,21 g (87%).

*Industrielle Anwendbarkeit*

Die mit Wasserstoffisotopen in der α-Stellung des Lactamringes markierten N-Vinyllactame verwendet man als Ausgangsverbindungen für die Synthese der Homo- und Copolymere. Die dabei gewonnenen hochmolekularen Substanzen können in der Medizin, beispielsweise als diagnostische Mittel, sowie für wissenschaftliche Forschungszwecke verwendet werden.

Die Herstellung markierter Homo- und Copolymere aus den erfindungsgemässen N-Vinyllactamen, die mit Wasserstoffisotopen in der α-Stellung des Ringes markiert sind, wird in an sich bekannter Weise, die für Polymerisation von Vinylmonomeren kennzeichnend ist, durchgeführt.

Die Anwendung der erfindungsgemässen Verbindungen zur Herstellung von mit Wasserstoffisotopen in der α-Stellung des Ringes markierten hochmolekularen Verbindungen hat folgende Vorteile:

— das mit Wasserstoffisotopen markierte Polymer wird durch ein konventionelles Verfahren, nämlich Radikalpolymerisation markierter Monomere, hergestellt;

— das erhaltene Polymer ist in einer genau festgesetzten Stellung stabil markiert;

— das Polymer enthält keine Radiolysenebenprodukte, es hat dieselbe molekulare Masse und dieselbe Molekularmassenverteilung, wie ein aus unaktivem Monomer hergestelltes Produkt;

— es kann ein Polymer mit beliebiger erforderlichen spezifischer Radioaktivität und beliebiger molekularer Masse erhalten werden.

Zur Bestätigung der Möglichkeit einer Anwendung der markierten Polymere in der Eigenschaft als diagnostische Mittel für medizinische Zwecke wird folgendes Beispiel angeführt: Bei 10 Ratten wurde mittels der gesamten Gammabestrahlung in einer Dosis von 2000 bis 2200 rad ein Syndrom der exsudativen Entheropathie hervorgerufen. Nach 48 Stunden nach der Bestrahlung führte man den Ratten intravenös je 0,3 ml einer 6%igen Lösung des in α-Stellung des Ringes mit Tritium markierten Poly-N-vinylpyrrolidons ($M_w$ = 60000, spezifische Radioaktivität 14 mCi/g) ein. 6 Stunden später wurden die Ratten getötet. Dann wurde der Dünndarm entfernt und dessen Inhalt nach spezieller Bearbeitung (Homogenisation) mit Hilfe eines Flüssigkeitsszintillationsspektrometers radiometrisch analysiert. Den Gehalt an markiertem Polymer im Inhalt des Dünndarms stellte man in Prozent zur eingeführten Menge der Lösung dar. Bei den Versuchstieren (10 Ratten) wurde die nach der Aktivität gemessene Menge des radioaktiven Poly-N-vinylpyrrolidons im Inhalt des Dünndarms im Vergleich gegenüber den Kontrolltieren (10 Ratten) dreimal grösser.

**Patentansprüche**

1. N-Vinyllactame, markiert mit den Wasserstoffisotopen in der α-Stellung des Lactamringes der allgemeinen Formel:

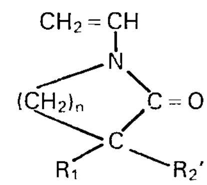

worin n = 2 bis 4; $R_1$ für H, D, T; $R_2$ für D, T steht.

2. Verfahren zur Herstellung der genannten Verbindungen nach Anspruch 1, dadurch gekennzeichnet, dass man das Ausgangs-N-Vinyllactam mit Deuteriumoxid bzw. mit tritiumenthaltendem Wasser bei einem pH-Wert von 10,5 bis 14 unter Anwendung als Katalysator eines Alkalimetall-hydroxides oder einer quaternären Base $NR_4OH$, worin R für $CH_3$ oder $C_2H_5$ steht, in Anwesenheit eines organischen Lösungsmittels oder ohne dieses umsetzt und anschliessend das Endprodukt isoliert.

**Claims**

1. N-vinyl lactams marked with the hydrogen isotopes in the α-position of the lactam ring of the general formula

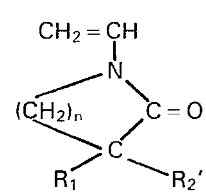

in which n = 2 to 4; $R_1$ represents H, D, T; $R_2$ represents D, T.

2. A method of obtaining the said compounds according to claim 1, characterized in that the starting N-vinyl lactam is reacted with deuterium oxide or with tritium-containing water respectively at a pH of from 10.5 to 14 while using as a catalyst an alkali metal hydroxide or a quaternary base $NR_4OH$, in which R represents $CH_3$ or $C_2H_5$, in the presence of

an organic solvant or without the latter, and the end product is then isolated.

**Revendications**

1. Lactames de N-vinyle marqués par les isotopes de l'hydrogène en position $\alpha$ du cycle du lactame de formule générale

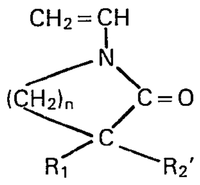

dans laquelle: n = 2 à 4; $R_1$ représente H. D. T; $R_2$ représente D, T.

2. Procédé de préparation des composés cités dans la revendication 1, caractérisé en ce que l'on traite le lactame de N-vinyle de départ avec de l'oxyde de deutérium ou avec de l'eau contenant du tritium à une valeur de pH de 10,5 à 14, en utilisant comme catalyseur un hydroxyde de métal alcalin ou une base quaternaire $NR_4OH$ dans laquelle R représente $CH_3$ ou $C_2H_5$, en présence ou en l'absence d'un solvant organique, et l'on isole ensuite le produit final.